# FASCICULE DE BREVET EUROPEEN

(11) **EP 1 010 417 B1**
(45) Date de publication et mention de la délivrance du brevet: **04.08.2004**
(21) Numéro de dépôt: 99403136.7
(22) Date de dépôt: 14.12.1999
(51) Int. Cl.: A61K 7/00, A61K 7/48

(54) **Solution d'un polymère du type polyacrylique et/ou polyvinylique associé à une charge et à un agent kératolytique et dispositif cosmétique de nettoyage et de soin**
Lösung von einem Polyacryl- und/oder Polyvinyl-Polymer mit einem Füllstoff und einem keratolytischen Agentien dazu, und kosmetische Vorrichtung zum Reinigen und Pflegen
Solution of a polyacryl and/or polyvinyl type polymer with a filler and a keratolytic agent and device for cleaning and care

(30) Priorité: 18.12.1998 FR 9816019
(43) Date de publication de la demande: 21.06.2000
(73) Titulaire: L'OREAL, 75008 Paris (FR)
(72) Inventeur: Gueret, Jean-Louis, 75016 Paris (FR)
(74) Mandataire: Tanty, François

(56) Documents cités:
- EP-A- 0 826 364
- WO-A-98/06375
- DE-A- 3 901 551
- FR-A- 2 734 574
- DATABASE WPI Section Ch, Week 9707 Derwent Publications Ltd., London, GB; Class A96, AN 97-073062 XP002109472 & JP 08 319213 A (TAIHEIYO KAGAKU KK), 3 décembre 1996 (1996-12-03)
- DATABASE WPI Section Ch, Week 9920 Derwent Publications Ltd., London, GB; Class A96, AN 99-232550 XP002109473 & JP 10 316560 A (TOKO YAKUHIN KOGYO KK), 2 décembre 1998 (1998-12-02)
- DATABASE WPI Section Ch, Week 8902 Derwent Publications Ltd., London, GB; Class A96, AN 89-013344 XP002109474 & JP 63 290817 A (SUNSTAR KK), 28 novembre 1988 (1988-11-28)
- PATENT ABSTRACTS OF JAPAN vol. 011, no. 115 (C-415), 10 avril 1987 (1987-04-10) & JP 61 260007 A (NITTO ELECTRIC IND CO LTD), 18 novembre 1986 (1986-11-18)

## Description

La présente invention a pour objet une solution incolore destinée à un usage cosmétique, contenant dans un milieu solvant organique cosmétiquement acceptable, au moins un polymère hydrophobe du type polyacrylique et/ou polyvinylique, au moins une charge, et au moins un agent kératolytique.

La présente invention a également pour objet l'utilisation de ladite solution pour la préparation d'un dispositif cosmétique destiné au nettoyage et au soin de la peau. Le dispositif selon l'invention est particulièrement efficace pour réaliser un nettoyage instantané de la peau par élimination mécanique et chimique des impuretés présentes à la surface de la peau du fait de la pollution, de la régénération cellulaire, de la transpiration ou de la sécrétion des glandes sébacées.

Selon un mode de réalisation préféré de la présente invention, la solution contient en outre au moins une substance cosmétiquement active, et peut être utilisée pour la préparation d'un dispositif destiné non seulement au nettoyage mais également au traitement de la peau grâce à la libération instantanée de la substance active simultanément à l'action de nettoyage. Le dispositif de nettoyage et de soin instantané de la présente invention se présente de préférence sous la forme d'un patch.

Il est déjà bien connu d'utiliser des patchs ou timbres à titre thérapeutique ou cosmétique qui sont constitués d'une structure composite comportant une ou plusieurs couches, ceux-ci étant appliqués sur la zone de peau à traiter, permettant ainsi la libération et la diffusion lente par transdermie d'une substance active présente dans une couche réservoir. De tels patchs présentent en effet une première couche dite couche support, généralement imperméable et occlusive afin de faciliter la transdermie, sur laquelle est fixée une seconde couche dite réservoir contenant la substance active, et également une couche adhésive soit sur la périphérie, soit sur toute la surface du patch, la protection étant assurée par une couche supérieure détachable. Ces patchs doivent être appliqués sur peau sèche et présentent une adhésivité croissante pendant la durée de l'application qui peut ainsi aller de plusieurs heures à plusieurs jours.

Ainsi, le brevet FR-A-2 738 744 décrit un patch pour la libération contrôlée d'au moins un composé actif instable en milieu oxydant. Ce patch comporte une couche

Ainsi, le brevet FR-A-2 738 744 décrit un patch pour la libération contrôlée d'au moins un composé actif instable en milieu oxydant. Ce patch comporte une couche support sur laquelle est fixée une couche réservoir anhydre constituée d'une matrice polymérique hydrophobe de silicone ou de polyuréthanne dans laquelle sont dispersées les particules d'un composé actif et d'au moins un agent hydroabsorbant. Ce type de patch a un pouvoir adhésif faible, c'est un patch traitant sans pouvoir nettoyant.

Le brevet US-A-5 026 552 décrit un patch contenant un polymère hydrogonflable qui s'applique sur une peau humide pendant 5 à 10 minutes et se retire d'un seul tenant lorsqu'il est sec, provoquant ainsi une action de peeling de la peau. Le polymère hydrogonflable décrit, est entre autres, l'alcool polyvinylique, les sels de métaux alcalins de carboxyméthylcellulose réticulé, les sels de métaux alcalins de l'acide polyacrylique, et les polyalkylènes glycols réticulés. Toutefois, ce patch ne permet la vectorisation d'un actif que s'il est laissé sur la peau pendant par exemple une nuit.

Ces patchs cosmétiques n'ont pas de pouvoir adhésif au temps zéro mais ne deviennent adhésifs qu'après leur humidification et séchage.

Il a par ailleurs été décrit dans les demandes de brevet EP-0 514 760 et EP-0 826 364, l'utilisation d'une composition à base de polymères substitués par des fonctions salifiables, et de solvant volatil tel que l'eau, l'éthanol ou l'alcool isopropylique, pour l'élimination des points noirs. Des pigments sont en outre incorporés dans la composition afin d'en améliorer l'efficacité. Les polymères constitutifs de la composition sont de préférence solubles dans l'eau mais peuvent également être hydrophobes et se présentent alors sous forme de dispersions ou d'émulsions. La composition s'applique sur la peau comme un cataplasme à l'aide d'un tissu de coton, de soie, de Nylon® ou même de plastique et est retirés après séchage. La composition peut en outre contenir une huile qui confère au film formé une certaine solidité évitant ainsi toute rupture lors de son retrait. Cette composition doit être appliquée sur une durée relativement longue d'au moins 30 minutes afin de réaliser un effet de peeling, mais ne permet pas la délivrance de substance active.

Il a également été proposé dans la demande internationale WO 93/05893 une composition constituée de polymères ayant des propriétés adhésives tels que le PVP, le mélange PVP/acétate de vinyle, et le mélange éther de méthylvinyle et acide maléique en milieu solvant volatil. La composition qui se présente sous forme d'un liquide ou d'un gel, est appliquée directement sur la peau, et sèche rapidement, en 3 à 10 minutes, pour former une couche solide élastique et pliable qui est retirée en appliquant un tissu ou un plastique adhésif. Il est toutefois souvent difficile de l'éliminer d'un seul tenant car celle-ci a tendance à se rompre facilement résultant en un peeling incomplet.

A la suite d'une sélection rigoureuse, on a constaté que certains polymères hydrophobes présentaient en eux-mêmes des propriétés satisfaisantes d'adhérence sur la peau et, qu'à partir d'une solution incolore de ces polymères dans un milieu solvant organique contenant au moins une charge et au moins un agent kératolytique, il était possible d'obtenir après évaporation du milieu solvant, des dispositifs nettoyants, notamment des patchs ou timbres, présentant une excellente auto-adhésivité.

La présente invention a donc pour objet, à titre de produit industriel nouveau, une solution incolore contenant, dans un milieu solvant organique cosmétiquement acceptable, (1) au moins un polymère hydrophobe auto-adhésif choisi parmi les polymères ou copolymères du type polyacrylique et/ou polyvinylique, (2) au moins une charge et/ou une huile essentielle, et (3) au moins un agent kératolytique, ledit polymère ayant, après évaporation dudit solvant organique, un pouvoir d'adhérence compris entre 150 et 800 g/cm².

Dans le cadre de la présente invention, on entend par solution incolore, une solution ne contenant pas de pigments ou de colorants et donc sans couleur particulière, ou avec une couleur blanchâtre ou crème.

Les polymères de la solution incolore selon la présente invention possèdent une structure linéaire et sont par ailleurs non ioniques et non hydrogonflables. Ils ont de préférence un poids moléculaire en masse compris entre 500.000 et 2.500.000 et de préférence 1.000.000 à 2.000 000.

Ces polymères sont généralement présents en une proportion comprise entre 20 et 60 % par rapport au poids total de la solution et possèdent, après évaporation du milieu solvant organique, un pouvoir d'adhérence sur la peau constant au cours du temps qui est compris entre 150 et 800 g/cm². Le pouvoir d'adhérence du ou des polymères correspond à la force exercée perpendiculairement au plan de la surface adhésive et qui est nécessaire à son décollement de la peau. Il est ainsi avantageux de prédéterminer la proportion en polymères dans la solution en fonction du pouvoir d'adhérence que l'on cherche à obtenir.

Les polymères selon l'invention, sont choisis de préférence parmi les copolymères du type acrylique/vinylique, et notamment les copolymères constitués de motifs dérivant de monomères choisis parmi les acrylates d'alkyle en C₁-C₂₀, l'acétate de vinyle, et l'acide acrylique et plus particulièrement d'acrylate d'éthylhexyle, d'acétate de vinyle, et d'acide acrylique.

Le milieu solvant organique cosmétiquement acceptable selon l'invention est par exemple constitué par des solvants tels que l'acétate d'éthyle, l'alcool éthylique, l'alcool isopropylique et leurs mélanges.

Le solvant organique est généralement présent en une proportion comprise entre 20 et 80 % du poids total de la solution.

On entend par charge toute substance propre à modifier la surface de la peau en très peu de temps, en apportant à la peau de la douceur. On peut notamment citer les poudres de nylon telles que le produit commercialisé par la Société ATOCHEM sous la dénomination ORGASOL® et les poudres de polyméthylméthacrylate telles que les produits commercialisés par la Société WACKHERR sous les dénominations COVABEAD®.

La charge et/ou l'huile essentielle est généralement présente en une proportion comprise entre 0,1 et 20 % et de préférence entre 0,5 et 10 % en poids par rapport au poids total de la composition

On entend par agent kératolytique, tout agent capable de nettoyer la peau de ses impuretés, en particulier des cellules mortes.

Tout agent kératolytique peut être utilisé dans les solutions de l'invention sans toutefois en affecter la nature ou le pouvoir ultérieur d'adhérence de celles-ci sur la peau

Ces agents sont bien connus dans l'industrie cosmétique et l'on peut citer, à titre d'exemples, les acides α-hydroxy carboxyliques tels que l'acide glycolique, l'acide lactique, l'acide tartrique, l'acide malique, l'acide citrique, l'acide mandélique, les acides β-hydroxy carboxyliques tels que l'acide salicylique mais également les acides de fruits, les acides β-cétocarboxyliques, leurs sels, amides ou esters. On peut en outre citer parmi ces agents l'acide kojique, le palmitate de retinyle et la vitamine C

De préférence, on utilise dans les compositions selon l'invention, des dérivés de l'acide salicylique tels que ceux décrits dans la demande de brevet EP-A-0 378 936. Parmi ceux-ci on peut citer, de façon non limitative, l'acide n-octanoyl-5-salicylique, l'acide n-décanoyl-5-salicylique et l'acide n-dodécanoyl-5-salicylique.

L'agent kératolytique est généralement présent en une proportion comprise entre 0,1 et 15 % en poids par rapport au poids total de la solution.

Selon un mode de réalisation préféré, la solution selon la présente invention contient en outre au moins une substance cosmétiquement active en une proportion comprise entre 1 et 15 % du poids total de la solution.

Les substances cosmétiquement actives susceptibles d'être incorporées dans la solution selon la présente invention peuvent être indifféremment du type hydrosoluble ou du type liposoluble et sont choisies, de manière générale, parmi les substances qui sont conventionnellement utilisées dans l'industrie de la cosmétique.

Selon un mode de mise en oeuvre avantageux de l'invention, les substances cosmétiquement actives sont dispersées de manière homogène dans la solution et peuvent se présenter sous forme solide, notamment sous forme particulaire.

Celles-ci peuvent être choisies parmi celles ayant une action hydratante, reconstituante, adoucissante, émolliente, cicatrisante, régénérante, astringente, matifiante, apaisante, auto-bronzante, raffermissante, déodorante, dépigmentante, éclaircissante, rafraîchissante ou nourrissante.

Celles-ci peuvent être également des agents aptes à absorber et/ou réguler le sébum et la transpiration, des agents liporégulateurs, des protecteurs solaires, des agents conditionneurs de la peau, des agents antirides, des agents tenseurs, des agents anti-radicaux libres, des agents anti-acnéiques, des désinfectants, des agents anti-vieillissement, des agents protecteurs vasculaires, des agents insensibilisants, des agents immunomodulateurs, des agents anti-inflammatoires, des agents antibactériens, ou des agents antifongiques et, bien sûr, l'association d'un ou plusieurs de ces agents.

Comme autres substances cosmétiquement actives, on peut citer par exemple le chitosane et ses dérivés, le tannin, la caféine, les huiles essentielles, le collagène, les filtres solaires, et l'acide hyaluronique.

La solution selon l'invention peut également contenir des particules d'au moins un agent hydro-absorbant dispersées de façon homogène dans ladite solution.

Parmi les agents hydro-absorbants présents à l'état dispersé au sein de la solution incolore selon l'invention, on peut citer, de préférence, les polyacrylates réticulés superabsorbants à fort taux de gonflement dans l'eau, tels que ceux commercialisés par la Société NORSOLOR sous la dénomination AQUAKEEP®, l'alcool polyvinylique, les polymères carboxyvinyliques tels que ceux commercialisés par la Société GOODRICH sous les dénominations de CARBOPOL®, les dérivés semi-synthétiques de la cellulose, tels que la carboxyméthylcellulose, les amidons, les biogommes telles que les gommes de xanthane, de guar, les gommes arabique et adragante, les biosaccharides, les scléroglucanes, la caséine, les phytocolloïdes, comme les alginates, les carragénates, l'agar-agar, la gélatine et les fibres de coton.

On préfère tout particulièrement utiliser les polyacrylates réticulés superabsorbants et les dérivés de la cellulose dont la présence à l'état dispersé favorise, après hydratation, sur la peau une meilleure disponibilité des particules des composés actifs présents.

L'agent hydro-absorbant tel que défini ci-dessus est présent, de préférence, en une proportion allant d'environ 0,2 % à environ 20 % en poids, et plus particulièrement allant de 0,5 % à 10 % par rapport au poids total de la solution.

La présente invention a également pour objet l'utilisation de la solution telle que décrite précédemment pour la réalisation d'un dispositif cosmétique auto-adhésif destiné au nettoyage et au soin de la peau.

Selon l'invention, le dispositif peut se présenter sous diverses formes telles que sous la forme d'un patch ou timbre, d'un rouleau ou sous toutes autres formes appropriées. Le dispositif comporte essentiellement un support qui peut être par exemple, sous la forme d'une surface plane, cylindrique, flexible, élastique ou rigide, d'épaisseur variable.

Selon un premier mode particulier de réalisation de l'invention, un patch est préparé extemporanément, par imprégnation ou revêtement d'un support à l'aide d'une quantité appropriée de la solution incolore selon l'invention, puis évaporation du milieu solvant. Le patch ainsi obtenu peut alors être appliqué directement sur la peau et se trouve maintenu par ses propriétés auto-adhésives.

Selon un deuxième mode de réalisation, un patch prêt à l'emploi est réalisé à partir d'un support que l'on enduit, selon la méthode classique de préparation des patchs, de la solution incolore selon l'invention et sur lequel, après évaporation du milieu solvant, on appose une feuille de revêtement protectrice détachable. Lors de l'utilisation, la feuille protectrice supérieure est enlevée, et le patch est alors appliqué sur la peau et y est maintenu par son pouvoir auto-adhésif.

Selon ces deux modes de réalisation, le patch est laissé sur la peau pendant un temps très court, compris entre environ 15 secondes et 20 minutes et de préférence entre 15 secondes et 10 minutes. Le patch est alors retiré, ce qui permet de réaliser en synergie un nettoyage mécanique et chimique de la peau grâce au pouvoir d'adhérence particulier des polymères ainsi qu'à la présence d'au moins un agent kératolytique.

Le support peut se présenter sous forme de feuilles, de films perforés ou non perforés, poreux ou non poreux, alvéolés ou non alvéolés, de trames tissées ou non tissées, de rouleaux ou de toutes autres pièces ayant une forme appropriée. Ceux-ci peuvent être indifféremment occlusifs ou non occlusifs.

A titre d'exemples, le support peut être constitué d'un matériau thermoplastique choisi parmi les polyéthylènes haute et basse densité, les polypropylènes, les polychlorures de vinyle, les copolymères d'éthylène et d'acétate de vinyle, les polyesters et les polyuréthannes, ou d'un complexe de tels matériaux.

Le support peut être de toute épaisseur appropriée qui procurera la fonction de support souhaitée. De préférence, l'épaisseur du support est comprise entre 20 µm et environ 1,5 mm. Avantageusement, la couche support est suffisamment flexible de manière à pouvoir épouser parfaitement le profil de la peau, et ne pas provoquer chez l'utilisateur, une sensation d'inconfort.

Lorsque le support est non occlusif, on utilise de préférence un support constitué d'un papier, d'un matériau thermoplastique poreux ou perforé, d'un matériau tissé, ou d'un matériau non tissé perforé.

La feuille protectrice détachable peut être par exemple une feuille de papier siliconée ou une feuille en matériau thermoplastique traitée pour la rendre anti-adhérente, par exemple à l'aide d'un vernis. De préférence, cette feuille protectrice détachable est en polyéthylène.

Lorsque les patchs ne se présentent pas sous forme prédécoupée, ceux-ci peuvent l'être selon un contour approprié correspondant à la surface de la peau à traiter, par exemple sous la forme d'un masque pour l'application sur le visage ou sous toute autre forme appropriée pour une application, notamment sur le nez, les joues, le contour des yeux, ou sur le front. En général, la taille des patchs est comprise entre 0,25 cm² et 500 cm², et de préférence, entre 1 cm² et 30 cm².

Les patchs ainsi constitués et découpés peuvent être utilisés, après élimination éventuelle de la couche protectrice détachable, par application sur la surface de la peau à traiter, ceux-ci se trouvant être parfaitement maintenus par les propriétés auto-adhésives des polymères polyvinyliques et/ou polyacryliques.

La présente invention a en outre pour objet un procédé cosmétique de nettoyage et de traitement rapide de la peau comprenant les étapes consistant :
(i) à appliquer sur un support tel que défini ci-dessus une quantité suffisante de la solution à base d'un polymère polyacrylique ou polyvinylique en milieu solvant ;
(ii) à laisser évaporer le solvant ;
(iii) à appliquer et à maintenir par auto-adhérence le support ainsi revêtu sur les parties de la peau à nettoyer et à traiter, pendant une durée comprise entre 15 secondes et 20 minutes ; et
(iv) à retirer le support par l'une de ses extrémités.

Alternativement, le procédé de nettoyage et de traitement comprend les étapes consistant :
(i) à détacher la feuille de revêtement protectrice d'un patch cosmétique de nettoyage et de traitement instantané prêt à l'emploi, tel que décrite ci-dessus,
(ii) à appliquer et à maintenir par auto-adhérence le patch sur les parties de la peau à nettoyer et à traiter pendant une durée comprise entre 15 secondes et 20 minutes ; et enfin
(iii) à retirer ledit patch.

De préférence, le temps de pose est compris entre 15 secondes et 5 minutes.

On va maintenant donner à titre d'illustration plusieurs exemples de solutions selon l'invention ainsi que des exemples de patchs et de leur utilisation pour le nettoyage et le traitement de la peau.

### EXEMPLES

### Exemple 1 :

On réalise une solution incolore comprenant en poids :
- 35 % de copolymère acrylate d'éthylhexyle/acétate de vinyle/acide acrylique de PM = 1.500.000,
- 52 % d'acétate d'éthyle,
- 3 % d'acide n-octanoyl-5-salicylique, et
- 10 % d'ORGASOL® commercialisé par la Société ATOCHEM.

Une feuille de polyéthylène perforée ayant la forme d'un masque et une épaisseur d'environ 30 µm, est imprégnée avec une quantité suffisante de la solution dont la formulation est donnée ci-dessus. Après évaporation de l'acétate d'éthyle, le patch ainsi obtenu est appliqué sur le visage, pour être ensuite retiré quelques minutes après. Après retrait du patch, on constate que la peau présente un aspect plus lisse et plus propre. Par ailleurs, la présence de l'ORGASOL® confère un aspect mat et naturel procurant également une agréable sensation de douceur.

### Exemple 2 :

On réalise une solution incolore comprenant en poids :
- 31 % de copolymère acrylate d'éthylhexyle/acétate de vinyle/acide acrylique de PM = 1.500.000,
- 46 % d'alcool isopropylique,
- 3 % d'acide n-octanoyl-5-salicylique,
- 10 % d'acide kojique, et
- 10 % d'ORGASOL® commercialisé par la Société ATOCHEM.

Un patch similaire à celui décrit dans l'Exemple 1 est réalisé à partir de la solution ci-dessus. Ce patch permet d'obtenir une exfoliation efficace de la peau tout en réalisant un éclaircissement très satisfaisant du visage en raison de la présence de l'acide kojique.

### Exemple 3 :

On réalise une solution incolore comprenant en poids :
- 37 % de copolymère acrylate d'éthylhexyle/acétate de vinyle/acide acrylique de PM = 1.500.000,
- 50 % d'alcool isopropylique,
- 3 % d'acide n-octanoyl-5-salicylique,
- 0,5 % d'huile essentielle de géranium,
- 0,5 % d'huile essentielle de Cédrat, et
- 9 % d' ORGASOL® commercialisé par la Société ATOCHEM.

Un patch constitué d'une trame non tissée, perforée, et alvéolée est préparé selon la méthode décrite dans l'Exemple 1. Ce patch est appliqué sur la partie de la peau à nettoyer pendant environ 15 secondes, et est ensuite retiré laissant ainsi une peau visiblement plus nette, plus souple et adoucie en raison du dépôt à sa surface de l'ORGASOL® et de l'action émolliente des huiles essentielles de géranium et de Cédrat.

## Revendications

1. Solution incolore à base d'un polymère hydrophobe dans un milieu solvant organique destinée à un usage cosmétique, **caractérisée par le fait qu'**elle contient dans ledit solvant organique cosmétiquement acceptable (1) au moins un polymère hydrophobe auto-adhésif, choisi parmi les polymères ou copolymères du type polyacrylique et/ou polyvinylique, (2) au moins une charge et/ou une huile essentielle, et (3) au moins un agent kératolytique, ledit polymère ayant, après évaporation dudit solvant organique, un pouvoir d'adhérence sur la peau compris entre 150 et 800 g/cm².

2. Solution selon la revendication 1, **caractérisée par le fait que** le polymère est constitué de motifs dérivant de monomères choisis parmi les acrylates d'alkyle en C₁-C₂₀, l'acétate de vinyle, et l'acide acrylique.

3. Solution selon la revendication 1, **caractérisée par le fait que** le polymère hydrophobe est présent en une proportion comprise entre 20 et 60 % du poids total de la solution.

4. Solution selon l'une quelconque des revendications précédentes, **caractérisée par le fait que** l'agent kératolytique est présent en une proportion comprise entre 0,1 et 15 % du poids total de la solution.

5. Solution selon l'une quelconque des revendications précédentes, **caractérisée par le fait que** le milieu solvant organique est présent en une proportion comprise entre 20 et 80 % du poids total de la solution.

6. Solution selon l'une quelconque des revendications précédentes, **caractérisée par le fait que** la charge et/ou l'huile essentielle est présente en une proportion comprise entre 0,1 et 20 % en poids par rapport au poids total de la composition.

7. Solution selon l'une quelconque des revendications précédentes, **caractérisée par le fait qu'**elle contient en outre une substance cosmétiquement active.

8. Solution selon la revendication 7, **caractérisée par le fait que** la substance cosmétiquement active est présente en une proportion comprise entre 1 et 15 % du poids total de la solution.

9. Solution selon l'une quelconque des revendications précédentes, **caractérisée par le fait qu'**elle contient en outre un composé hydro-absorbant.

10. Utilisation de la solution selon l'une quelconque des revendications précédentes pour l'obtention d'un dispositif destiné au nettoyage et au soin instantané de la peau.

11. Utilisation selon la revendication 10, **caractérisée par le fait que** ledit dispositif est un patch formé d'un support qui a été revêtu de la solution selon les revendications 1 à 9.

12. Utilisation selon les revendications 10 et 11, **caractérisée par le fait que** ledit support se présente sous la forme d'une feuille ou d'un film tissé ou non, perforé ou non, occlusif ou non, poreux ou non, alvéolé ou non.

13. Utilisation selon les revendications 10 à 11, **caractérisée par le fait que** ledit support se présente sous la forme d'un cylindre.

14. Utilisation selon l'une quelconque des revendications 10 à 13, dans laquelle ledit dispositif destiné au nettoyage de la peau comprend en outre une feuille protectrice détachable supérieure.

15. Patch destiné au nettoyage et au soin instantané de la peau comprenant un support imprégné d'une quantité suffisante de la solution selon les revendications 1 à 9.

16. Procédé cosmétique de nettoyage de la peau comprenant les étapes consistant :
(i) à appliquer sur un support une quantité suffisante de la solution selon les revendications 1 à 9 ;
(ii) à laisser évaporer le solvant ;
(iii) à appliquer le support ainsi revêtu sur les parties de la peau à nettoyer pendant une durée comprise entre environ 15 secondes et 20 minutes ; et
(iv) à retirer le support par l'une de ses extrémités.

## Patentansprüche

1. Farblose Lösung auf Basis eines hydrophoben Polymers in einer organischen Lösungsmittelumgebung, die für eine kosmetische Verwendung vorgesehen und **dadurch gekennzeichnet ist, dass** in dem besagten, kosmetisch annehmbaren Lösungsmittel (1) mindestens ein selbstklebendes hydrophobes Polymer, ausgewählt unter Polyacryl- und/oder Polyvinylpolymeren oder -copolymeren, (2) mindestens ein Zuschlag und/oder ein essentielles Öl, und (3) mindestens ein keratolytisch wirkendes Mittel enthalten ist, wobei das besagte Polymer nach dem Verdampfen des besagten organischen Lösungsmittels ein Haftungsvermögen auf der Haut zwischen 150 und 800 g/cm² aufweist.

2. Lösung nach Anspruch 1, **dadurch gekennzeichnet, dass** sich das Polymer aus Motiven zusammengesetzt, abgeleitet von Monomeren, ausgewählt unter C₁-C₂₀ Alkylacrylaten, Vinylacetat oder Acrylsäure.

3. Lösung nach Anspruch 1, **dadurch gekennzeichnet, dass** das hydrophobe Polymer in einem Anteil zwischen 20 und 60 % des Gesamtgewichts der Lösung vorhanden ist.

4. Lösung nach einem der vorhergehenden Ansprüche, **dadurch gekennzeichnet, dass** das keratolytisch wirkende Mittel in einem Anteil zwischen 0,1 und 15 % des Gesamtgewichts der Lösung vorhanden ist.

5. Lösung nach einem der vorhergehenden Ansprüche, **dadurch gekennzeichnet, dass** die organische Lösungsmittelumgebung in einem Anteil zwischen 20 und 80 % des Gesamtgewichtes der Lösung vorhanden ist.

6. Lösung nach einem der vorhergehenden Ansprüche, **dadurch gekennzeichnet, dass** der Zuschlag und/oder das essentielle Öl in einem Anteil zwischen 0,1 und 20 Gew.-%, bezogen auf das Gesamtgewicht der Zusammensetzung, vorhanden ist.

7. Lösung nach einem der vorhergehenden Ansprüche, **dadurch gekennzeichnet, dass** sie zusätzlich eine kosmetisch aktive Substanz enthält.

8. Lösung nach Anspruch 7, **dadurch gekennzeichnet, dass** die kosmetisch aktive Substanz in einem Anteil zwischen 1 und 15 % des Gesamtgewichtes der Lösung vorhanden ist.

9. Lösung nach einem der vorhergehenden Ansprüche, **dadurch gekennzeichnet, dass** sie zusätzlich eine feuchtigkeitsabsorbierende Verbindung enthält.

10. Verwendung der Lösung nach einem der vorhergehenden Ansprüche, um eine Vorrichtung zu erhalten, die für die Reinigung und die sofortige Pflege der Haut vorgesehen ist.

11. Verwendung nach Anspruch 10, **dadurch gekennzeichnet, dass** die besagte Vorrichtung ein Pflaster ist, das aus einem Träger gebildet ist, der mit der Lösung nach den Ansprüchen 1-9 behandelt wurde.

12. Verwendung nach den Ansprüchen 10 und 11, **dadurch gekennzeichnet, dass** der besagte Träger als Bogen oder als gewebter / nicht gewebter, perforierter / nicht perforierter, verschlossener / nicht verschlossener, poröser / nicht poröser, zellenartiger / nicht zellenartiger Film vorliegt.

13. Verwendung nach den Ansprüchen 10 bis 11, **dadurch gekennzeichnet, dass** der besagte Träger in zylindrischer Form vorliegt.

14. Verwendung nach einem der Ansprüche 10 bis 13, in dem die für die Reinigung der Haut vorgesehene Vorrichtung zusätzlich einen oberen, abtrennbaren, schützenden Bogen umfasst.

15. Pflaster, das zur Reinigung und sofortigen Pflege der Haut vorgesehen ist und das einen Träger umfasst, der mit einer ausreichenden Menge der Lösung nach Ansprüchen 1 bis 9 imprägniert wurde.

16. Kosmetisches Verfahren zur Reinigung der Haut, das folgende Schritte umfasst, bestehend aus:
(i) der Anwendung einer ausreichenden Menge der Lösung nach Ansprüchen 1 bis 9 auf einen Träger;
(ii) dem Verdampfen-lassen des Lösungsmittels;
(iii) der Anwendung des so behandelten Trägers auf den zu reinigenden Partien der Haut für eine Zeit zwischen 15 Sekunden und 20 Minuten; und
(iv) dem Abziehen des Trägers an einem seiner Enden.

## Claims

1. A colorless solution for cosmetic use, based on a hydrophobic polymer in an organic solvent medium, which contains, in said cosmetically acceptable organic solvent, (1) at least one self-adhesive hydrophobic polymer selected from polymers or copolymers of the polyacrylic and/or polyvinylic type, (2) at least one filler and/or essential oil, and (3) at least one keratolytic agent, said polymer having an adhesiveness on the skin of between 150 and 800 g/cm² after evaporation of said organic solvent.

2. A solution as claimed in claim 1 wherein the polymer consists of units derived from monomers selected from C₁-C₂₀-alkyl acrylates, vinyl acetate and acrylic acid.

3. A solution as claimed in claim 1 wherein the hydrophobic polymer is present in a proportion of between 20 and 60% of the total weight of the solution.

4. A solution as claimed in any one of the preceding claims wherein the keratolytic agent is present in a proportion of between 0.1 and 15% of the total weight of the solution.

5. A solution as claimed in any one of the preceding claims wherein the organic solvent medium is present in a proportion of between 20 and 80% of the total weight of the solution.

6. A solution as claimed in any one of the preceding claims wherein the filler and/or essential oil are present in a proportion of between 0.1 and 20% by weight, based on the total weight of the composition.

7. A solution as claimed in any one of the preceding claims which also contains a cosmetically active substance.

8. A solution as claimed in claim 7 wherein the cosmetically active substance is present in a proportion of between 1 and 15% of the total weight of the solution.

9. A solution as claimed in any one of the preceding claims which also contains a water-absorbing compound.

10. Use of the solution as claimed in any one of the preceding claims for the preparation of a device for instantaneous cleansing and care of the skin.

11. Use as claimed in claim 10 wherein said device is a patch formed of a support which has been coated with the solution as claimed in claims 1 to 9.

12. Use as claimed in claims 10 and 11 wherein said support takes the form of a sheet or a film which is woven or non-woven, perforated or non-perforated, occlusive or non-occlusive, porous or non-porous and alveolate or non-alveolate.

13. Use as claimed in claims 10 and 11 wherein said support takes the form of a cylinder.

14. Use as claimed in any one of claims 10 to 13 wherein said skin cleansing device also comprises a detachable top protective sheet.

15. A patch for instantaneous cleansing and care of the skin, comprising a support impregnated with a sufficient amount of the solution as claimed in claims 1 to 9.

16. A cosmetic method of cleansing the skin, comprising steps consisting in:
(i) applying, to a support, a sufficient amount of the solution as claimed in claims 1 to 9;
(ii) leaving the solvent to evaporate;
(iii) applying the coated support to the parts of the skin to be cleansed, for a period of between about 15 seconds and 20 minutes; and
(iv) removing the support by one of its ends.
